# EUROPEAN PATENT SPECIFICATION

(11) **EP 1 034 812 B1**
(45) Date of publication and mention of the grant of the patent: **10.05.2006**
(21) Application number: 00104535.0
(22) Date of filing: 13.03.2000
(51) Int. Cl.: A61M 27/00

(54) **Hydrophobic vent incorporated into cerebral spinal fluid drainage chamber**
In eine Drainagekammer für Cerebrospinalflüssigkeit eingebaute hydrophobe Entlüftung
Event hydrophobe incorporé dans une chambre de drainage de fluide cérébrospinal

(30) Priority: 11.03.1999 US 266674
(43) Date of publication of application: 13.09.2000
(73) Proprietor: MEDTRONIC, INC., Minneapolis, Minnesota 55432-5604 (US)
(72) Inventor: Harper, Derek J., Santa Ynez, CA 93460 (US); Vasek, Jeffrey A., Golela, CA 93117 (US); Vaskelis, Paul S., Santa Jose, CA 95123 (US)
(74) Representative: Hughes, Andrea Michelle

(56) References cited:
- US-A- 3 776 229
- US-A- 4 184 484
- US-A- 4 465 479
- US-A- 4 621 647
- US-A- 5 462 667
- US-A- 5 772 625
- US-A- 5 779 674

## Description

This invention relates to a device for draining excess cerebral spinal fluid from a patient's brain and more particularly relates to a device for venting a rigid drip chamber used to drain excessive cerebral spinal fluid (CSF) from a patient's brain.

A typical adult has a total of about 120 - 150 cc of CSF with about 40 cc in ventricles in the brain. A typical adult also produces about 400 - 500 cc/day of CSF, all of which is reabsorbed into the blood stream on a continuous basis. CSF is comprised primarily of water but also includes small amounts of minerals and proteins. Occasionally, blood is present in CSF. This often occurs with trauma to the brain or as a result of other conditions that cause hydrocephalus.
Sometimes, the brain produces excess CSF. Two common causes of the excess production of CSF are hydrocephalus and brain trauma although there are many other causes. Hydrocephalus is a condition of excessive accumulation of CSF in the ventricles or brain tissue. Hydrocephalus can result from genetic conditions or from trauma to the brain. Brain trauma often results from head injuries or other accidents.

Excessive accumulation of CSF, due to hydrocephalus, brain trauma or other causes, manifests itself as increased pressure within the brain. Whatever the cause, over time, this increased CSF pressure causes damage to the brain tissue. It has been found that relieving the CSF pressure is therapeutically beneficial. This is usually done by draining CSF from the ventricles.

Patients with hydrocephalus or head trauma often continue to produce excess CSF, at least over some time period. Therefore, it is often desirable to continuously drain excess CSF to maintain normal CSF pressure in the brain.

US 5,779,674 discloses a drip chamber for fluid gas removal having a hydrophobic barrier to avoid formation of an air space at the top of the drip chamber.

US 4,621,647 describes an apparatus for monitoring and regulating intracranial pressure.

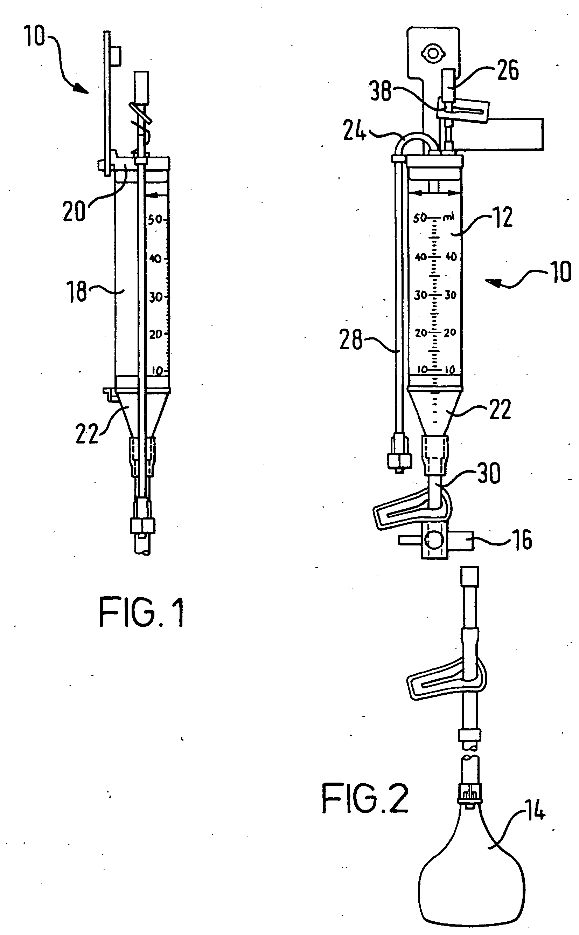

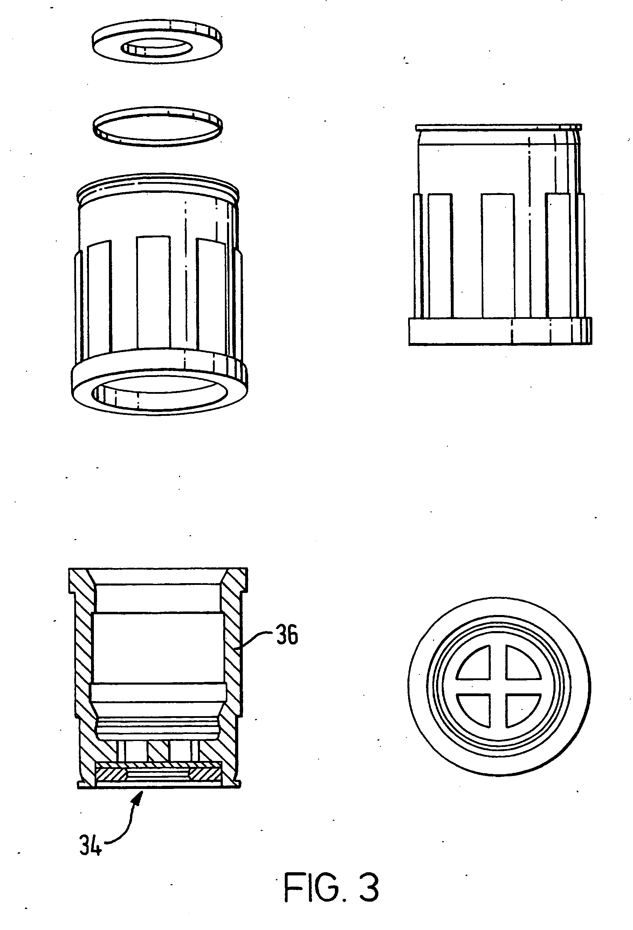

Examples of systems to continuously drain excess CSF are the Becker System® and the EDM Drainage System® made and sold by Medtronic - PS Medical of Goleta, California. Such a device is shown in Figures 1 through 3. In the Figures, the drainage system is shown generally labeled 10. The system includes a rigid drip chamber 12 and a drainage bag 14. A stopcock 16 connects drip chamber 12 to drainage bag 14.

Drip chamber 12 is made of a generally cylindrical, rigid tube 18, an inlet manifold 20 and an outlet manifold 22. Inlet manifold 20 has an inlet 24 and a vent 26. Inlet 24 is connected through tubing 28 to a ventricular catheter (not shown) placed in a patient's head that drains CSF from the patient's ventricle. Outlet manifold 22 has an outlet 30 that is connected to stopcock 16.

Vent 26 is formed by placing a porous material 32 across an opening 34 in a housing 36. Housing 36 is connected to inlet manifold 20 through a connecting tube 38. In this way, vent 26 is fluidly connected to drip chamber 12 through connecting tube 38.

Another example of a system to continuously drain excess CSF is shown in US Patent No. 4,731,056 issued to William S. Tremulis on March 15, 1988 and entitled "External Drainage Antisiphon Device." A further such system is disclosed in US Patent No. 5,772,625 issued to John A. Krueger, Kevin M. Jaeger and Helmut W. C. Rosenberg on June 30, 1998 and entitled "External Drainage Shunt."

All of these prior art systems have rigid chambers to collect and measure the accumulation of CSF drained from the patient. In these prior art systems, it is necessary to vent the rigid drip chambers at the top of the chamber to allow the passage of air in and out of the chamber as cerebrospinal fluid (CSF) moves in and out.

Patients with hydrocephalus that are being treated by draining excess CSF through the use of a drainage system including a drip chamber are often moved from place to place. During movement, the drip chamber is often moved from its substantially vertical alignment and is laid flat in a substantially horizontal orientation.

One current industry practice attempts to prevent contact of the vent by CSF by placing the vent physically away from the inside of the chamber. The venting is typically accomplished by placing a vent on the outside of the top of the chamber. The vent 26 is typically fluidly connected to the inside of the drip chamber 12 through a relatively narrow tube or channel. When the system is laid flat, CSF may enter the tube or channel that connects the vent 26 to the inside of the drip chamber 12. Then, when the system is raised to vertical, the CSF may not drain from the tube or channel. Consequently, an "airlock" is formed. As a result, air often cannot leave or enter the drip chamber 12.

Another cause of preventing air from moving in or out of the drip chamber 12 occurs in systems where the vent 26 is attached directly to drip chamber 12 but is made of a hydrophilic material or a material that is not sufficiently hydrophobic. The vent 26 being "hydrophilic" means that the vent is made of material that preferentially absorbs water. Not being sufficiently hydrophobic means that the material, while exhibiting some tendency to resist water, never the less has some tendency to attract and retain water.

A major component of CSF is water. In a vent that is made of hydrophilic material or material that is not sufficiently hydrophobic, when the CSF comes into contact with the vent 26 as a result of the drip chamber 12 being laid on its side or being agitated, the CSF is absorbed into and "plugs" the vent 26. Plugging the vent means that air cannot pass across the vent so that the "venting" function cannot be accomplished.

This lack of air movement across the vent 26 can cause two problems depending on whether the air is blocked from moving into or out of the drip chamber 12. If air is prevented from moving out of the drip chamber 12, an "effective airlock" is formed resulting in underdrainage of CSF. If air is prevented from moving into the drip chamber 12 as CSF is drained out of drip chamber 12 into drainage bag 14, a siphon effect will be created leading to overdrainage of CSF.

When water contacts a hydrophilic material or a material not sufficiently hydrophobic, a condition called "wetting" results. This means that air is prevented from flowing through the material. Components of CSF in addition to water reduce the surface tension of the fluid. This compounds the "wetting" effect and makes the vent 26 more likely to be plugged by contact with the CSF than it would be with contact with water alone. This "wetting" problem is even more compounded when the CSF that contacts the vent has blood in it. The blood acts as a surfactant which increases the plugging of the material of the vent. As a result, even a small amount of blood in the CSF can cause the vent to plug rapidly if it contacts the hydrophilic vent material.

If air cannot leave the chamber, any additional fluid entering the chamber must compress the air already in the chamber. As a result, the CSF pressure inside the patient's head must increase beyond desired levels to continue to push the fluid into the chamber. The increased cranial pressure results in underdrainage of CSF and can lead to coma and death.

Another problem occurs when air cannot enter the drip chamber 12. In operation, the stopcock 16 is often closed while draining CSF from the brain so that the amount of CSF drained can be accurately measured in the drip chamber 12. After the CSF has been measured, the stopcock 16 is opened and the CSF drains from drip chamber 12 into the drainage bag 14. If air cannot enter the drip chamber 12 because the vent 26 is plugged, pressure in drip chamber 12 will be reduced as the CSF drains out. As a result, a "siphon" will be created in the tubing 28 as the CSF drains into the drainage bag 14. This siphon will cause additional CSF in the patient's brain to be drained resulting in overdrainage of CSF. Overdrainage of CSF can lead to subdural hematoma and possibly death.

In addition, moving the vent 26 physically away from the drip chamber 12 to prevent contact with CSF, some current systems try to prevent contact between the vent 26 and CSF by having warnings against exposing the drip chamber vent to CSF fluid. Further, some drainage systems provide manually operated shut-offs, such as stopcocks, between the drip chamber 12 and vent 26 to prevent CSF from moving into contact with the vent during transport or at other times when the system is positioned horizontally.

Some current industry devices have vents made of either hydrophilic material or material that is not sufficiently hydrophobic to prevent wetting and plugging.

The present invention provides a drip chamber for draining cerebral spinal fluid (CSF) from a brain comprising: a tube; an outlet manifold in fluid communication with the tube, the outlet manifold having an outlet; an inlet manifold in fluid communication with the tube, the inlet manifold having an inlet and an outer surface, the inlet manifold having a vent, the vent including a porous material; characterised in that the pore size of the porous material is from .22 µm to 5.0 µm and wherein the vent has a surface area of from 0.8 cm² to 5.0 cm²; and further characterised by the porous material being adhered to the inside surface of the inlet manifold.

The preferred embodiment comprises several features which are each independently useful or which may be combined in a variety of combinations. One preferred feature of the invention is placing an atmospheric reference vent at or near the top of a rigid drip chamber for draining CSF from a patient. In the preferred embodiment, the vent is placed on the inside of the drip assembly, immediately next to the CSF. Making the vent integral with the top of the drip chamber eliminates the tube or channel that was present in the prior art devices. This configuration produces a vent that withstands CSF exposure without forming an "airlock" and the corresponding compromised venting capability.

The vent, in another embodiment of the invention, is made of a very hydrophobic material, expanded polytetraflouroethylene (e-PTFE). The problem of a hydrophilic vent or a not sufficiently hydrophobic vent getting wet and thereby becoming essentially "blocked" is solved in the present invention by using a very hydrophobic material that eliminates the possibility of blocking due to "wetting" when exposed to CSF or bloody CSF at physiologically significant concentrations and pressures.

In yet another embodiment of the invention, the vent is made of a porous material having a pore size that allows air to readily pass through while preventing CSF from passing through. A preferred embodiment includes making the vent of expanded polytetraflouroethylene (e-PTFE) with a pore size ranging from about 0.22 µm to about 5.0 µm and more preferable a pore size of about 3 µm. The larger the pore size, the smaller the surface area needs to be to allow adequate venting of air from the drip chamber through the vent. The converse is also true so that vents with smaller pore sizes will need to have larger surface area to allow adequate venting.

In another embodiment of the invention, the vent material is made of a porous material that also prevents microbes from passing through the vent material into the drip chamber. If microbes get into the drip chamber, it is feared that the microbes could pass out of the drip chamber "upstream" through the tubing to the patient's brains with potentially serious adverse effects. Preventing microbes from entering the drip chamber through the vent material helps to prevent microbial access to the patient's brain. The material of the vent has a pore size to allow air to pass through it but prevent CSF fluid from passing out of the drip chamber through the material and prevent microbes from entering the drip chamber through the material.

The present invention makes it easier for patients with a need to drain CSF to be treated while they are being transported. With the invention, the CSF drainage system may be laid down horizontally for transport of the patient. If CSF is in the drip chamber, it will not adversely affect the venting properties of the filter when the system is raised to vertical.

The present invention also has another therapeutic advantage. Because the filter will not be fouled by exposure to CSF or bloody CSF, the vent will always allow the drip chamber to be vented to atmosphere. This will allow the system to have accurate pressure settings for the drainage of CSF from patient.

It is therefore a primary objective of the invention to produce a vent that will not produce an "airlock" if the drip chamber is moved from its substantially vertical orientation to a substantially horizontal orientation.

It is another primary objective of the invention to produce a vent that will not be "plugged" or "fouled" by contact with CSF.

It is a further primary objective of the invention to produce a vent that performs its venting function while preventing the ingress of microbes through the vent.

These and other objectives of the invention will be clear to those skilled in the art from the description, which is given by way of example only, of preferred embodiments of the invention set out herein and particularly with reference to the following Detailed Description of the Invention and the corresponding drawings. In the drawings, like elements, wherever shown, are referenced by like reference numbers.
Figure 1 is a front view of a prior art CSF drainage system.
Figure 2 is a side view of the system of Figure 1.
Figure 3 is a side cross-sectional view of the vent of the system of Figures 1 and 2.
Figure 4 is a perspective view of a system incorporating the present invention.

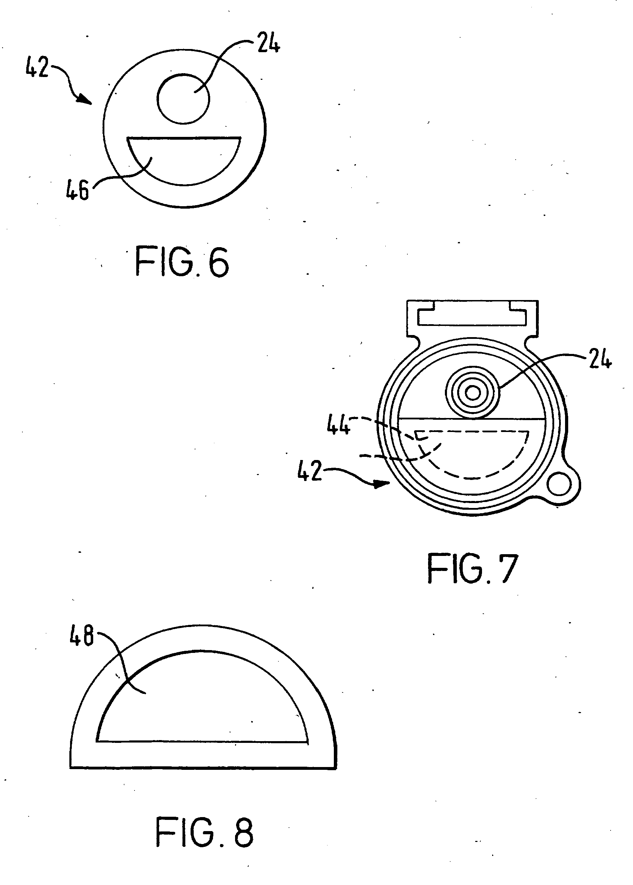

Figure 5 is an exploded perspective view of the drip chamber of the system of Figure 4.
Figure 6 is a top view of the inlet manifold of the drip chamber of the system of Figure 4 with the vent in place.
Figure 7 is a top view of the inlet manifold of the drip chamber of the system of Figure 4 without the vent.
Figure 8 is a top view of the vent of the system of Figure 4.

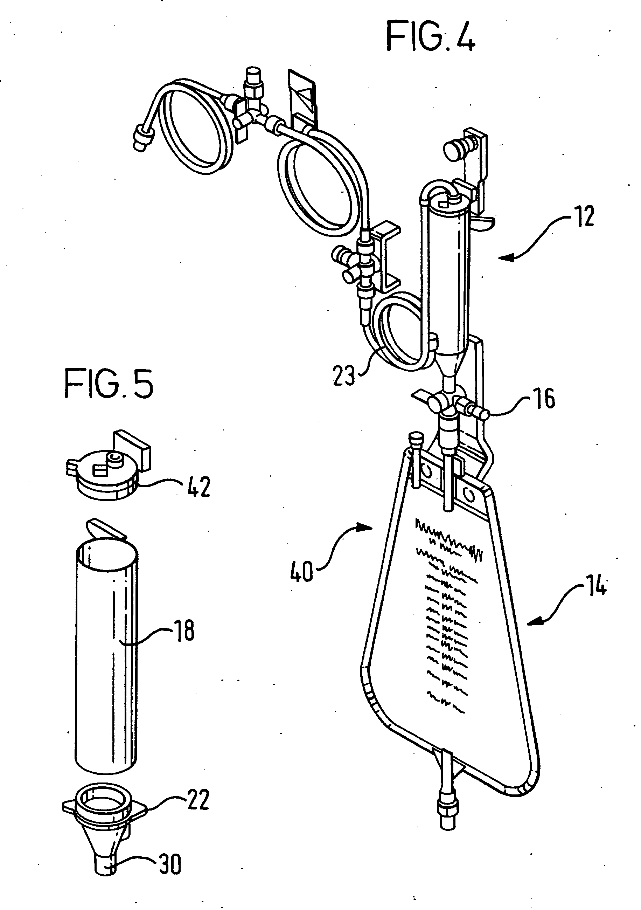

A CSF drainage system incorporating the present invention is shown in Figure 4 generally labeled 40. The system includes a rigid drip chamber 12 and a drainage bag 14. A stopcock 16 connects drip chamber 12 to drainage bag 14.

As mentioned, drip chamber 12 is made of a generally cylindrical, rigid tube 18 and includes an outlet manifold 22. Drip chamber 12 also has an inlet manifold 42 that is slightly different from inlet manifold 20. Inlet manifold 42 also has an inlet 24 but has a vent 44 that is different than vent 26 as will be described hereafter. Inlet 24 is connected through tubing 28 to a shunt (not shown) placed in a patient's head that drains CSF from the patient's ventricle. Outlet manifold 22 has an outlet 30 that is connected to stopcock 16.

Vent 44 is formed by covering a hole 46 in inlet manifold 42 with a porous, hydrophobic material 48. Material 48 is adhered to the inside surface of the inlet manifold 42. In the preferred embodiment, material 48 is made of expanded polytetraflouroethylene (ePTFE). The preferred pore size for material 48 ranges from about 0.22 µm to about 5.0 µm and more preferably has a pore size of about 3 µm. This range of pore sizes allows air molecules to pass through the vent 44 while preventing water or CSF molecules to pass through the vent 44. In addition, this pore size prevents microbes from passing through material 48 into drip chamber 12.

The preferred material for adhering material 48 to inlet manifold 42 is a biocompatible adhesive such as is well understood in the art. Of course, other adhesives, though less desirable, could also be used as well. In addition, other means of adhering the vent to the inlet manifold, including but not limited to a heat staking, ultrasonic welding or RF welding could be used as well. ePTFE is hydrophobic. In fact, it is the most hydrophobic porous material now known. As a result, it is the most resistive to clogging and loss of low pressure venting properties due to exposure of CSF or CSF with Blood.

In view of the use of the drip chamber 12, the filter must maintain venting properties when fluid is infused into the drip chamber at a nominal rate of 20 ml/hr. In extreme conditions, it may be necessary to vent up to 100 ml/hr. This will allow the drip chamber 12 to properly vent under extreme use conditions. Extreme conditions means that the vent 44 is in contact with CSF containing blood and under high flow rates. In order to properly vent under even extreme use conditions, resistance to CSF flow may not exceed 2.2 cm of H2O as measured by including the resistance to CSF flow due to resistance in the tubing 28. With the range of pore sizes needed to selectively pass air molecules while inhibiting the passage of water and CSF.

As mentioned above, the blood in CSF is the most severe challenge to the operation of the vent 44. However, the vent 44 should have a pore size sufficient to prevent intrusion of CSF when the drip chamber 12 is internally pressurized to 50 mm of Hg. It is important that the surface area of vent 46 be sufficiently large to still allow adequate venting of drip chamber 12 even if the material 48 of vent 44 has come in contact with CSF containing blood. We have found that for ePTFE material having a pore size of about 3 µm, a surface area greater than about .8 cm² will allow proper venting of the drip chamber 12 under even extreme conditions.

In another embodiment of the invention, the porous material 32 of vent 26 shown in Figures 1,2 and 3 and described above is replaced with ePTFE as described above with the corresponding pore size and surface areas also described above.

## Claims

1. A drip chamber (12) for draining cerebral spinal fluid (CSF)from a brain comprising:
a tube (18);
an outlet manifold (22) in fluid communication with the tube, the outlet manifold (22) having an outlet (30);
an inlet manifold (42) in fluid communication with the tube (18), the inlet manifold (42) having an inlet (24) and an outer surface, the inlet manifold (42) having a vent (44), the vent (44) including a porous material (48); wherein the pore size of the porous material is from 22 µm to 5.0 µm **characterised in that** the vent (44) has a surface area of from 0.8 cm² to 5.0 cm²: and further **characterised by** the porous material (48) being adhered to the inside surface of the inlet manifold (42).

2. The drip chamber (12) of claim 1 wherein the pore size of the porous material (48) is from .45 µm to 5.0 µm.

3. The drip chamber (12) of any of the preceding claims wherein the pore size of the porous material (48) is about 3 µm.

4. The drip chamber (12) of any of the preceding claims wherein the porous material (48) is expanded polytetraflouroethylene (e-PTFE).

5. The drip chamber (12) of any of the preceding claims wherein the porous material (48) is a hydrophobic material.

6. The drip chamber (12) of any one of the preceding claims wherein the tube (18) is rigid.

7. The drip chamber (12) of any one of the preceding claims wherein the tube (18) is generally cylindrical.

8. The drip chamber (12) of any of the preceding claims wherein the porous material (48) is formed in the inlet manifold (42) by creating a hole in the inlet manifold (42) and covering the hole with the porous material (48).

9. The drip chamber (12) of claim 7 wherein the porous material (48) is adhered to the inside surface of the inlet manifold (42) by a technique chosen from the group consisting of biocompatible adhesive, heat staking, ultrasonic welding or radio frequency (RF) welding.

10. A drip chamber system for draining cerebral spinal fluid (CSF) from a brain, comprising a drip chamber (12) as claimed in any preceding claim;
a drainage bag (14); and
a stopcock connecting the tube (18) to the drainage bag (14) through the outlet.

## Patentansprüche

1. Drainage- bzw. Tropfkammer (12) zur Drainage von Cerebrospinalflüssigkeit (CSF) von einem Gehirn, mit:
einem Rohr bzw. Röhrchen (18);
einem Ausgangs-Übergangsstück (22) in fluider Kommunikation mit dem Rohr, wobei das Auslass- Übergangsstück (22) einen Auslass (30) aufweist;
einem Einlass-Übergangsstück (42) in fluider Kommunikation mit dem Rohr (18), wobei das Einlass-Übergangsstück (42) einen Einlass (24) und eine äußere Oberfläche aufweist, wobei das Einlass-Übergangsstück (42) eine Entlüftung bzw. einen Luftdurchlass (44) aufweist, wobei der Luftdurchlass (44) ein poröses bzw. poriges Material (48) umfasst, wobei die Porengröße des porigen Materials von 0,22 µm bis 5,0 µm beträgt, **dadurch gekennzeichnet, dass** der Luftdurchlass (44) einen Oberflächenbereich von 0,8 cm² bis 5 cm² aufweist, und ferner **dadurch gekennzeichnet, dass** das porige Material (48) an der inneren Oberfläche des Einlass-Übergangsstücks (42) anhaftet bzw. mit diesem verklebt ist.

2. Drainagekammer (12) nach Anspruch 1, bei der die Porengröße des porigen Materials (48) von 0,45 µm bis 5,0 µm beträgt.

3. Drainagekammer (12) nach einem der vorstehenden Ansprüche, bei der die Porengröße des porigen Materials (48) in etwa 3 µm beträgt.

4. Drainagekammer (12) nach einem der vorstehenden Ansprüche, bei der das porige Material (48) expandiertes Polytetrafluoroethylen (e-PTFE) ist.

5. Drainagekammer (12) nach einem der vorstehenden Ansprüche, bei der das porige Material (48) ein hydrophobes Material ist.

6. Drainagekammer (12) nach einem der vorstehenden Ansprüche, bei der das Rohr (18) starr ausgebildet ist.

7. Drainagekammer (12) nach einem der vorstehenden Ansprüche, bei der das Rohr (18) in allgemeinen bzw. im wesentlichen zylindrisch ausgebildet ist.

8. Drainagekammer (12) nach einem der vorstehenden Ansprüche, bei der das porige Material (48) in dem Einlass-Übergangsstück (42) gebildet ist bzw. wird durch Erzeugung eines Loches in dem Einlass-Übergangsstück (42) und Bedecken bzw. Abdecken des Loches mit dem porigen Material (48).

9. Drainagekammer (12) nach Anspruch 7, bei der das porige Material (48) mit der inneren Oberfläche des Einlass-Übergangsstückes (42) verklebt ist mittels einer Technik, die ausgebildet ist aus der Gruppe, die besteht aus biokompatibler Verklebung bzw. biokompatiblem Kleber, nietenartige Fügung bzw. Heißverprägung, Ultraschallschweißen oder Radiofrequenzschweißen bzw. RF-Schweißen.

10. Drainagekammersystem zur Drainage von cerebrospinaler Flüssigkeit (CSF) von einem Gehirn, mit einer Drainagekammer (12) nach einem der vorstehenden Ansprüche;
einem Drainagebeutel (14); und
einem Absperrelement, das das Rohr (18) über den Auslass mit dem Drainagebeutel (14) verbindet.

## Revendications

1. Chambre compte-gouttes (12) pour drainer du fluide cérébrospinal (CSF) à partir d'un cerveau, comportant :
un tube (18) ;
un collecteur de sortie (22) en communication hydraulique avec le tube, le collecteur de sortie (22) ayant une sortie (30) ;
un collecteur d'entrée (42) en communication de fluide avec le tube (18), le collecteur d'entrée (42) ayant une entrée (24) et une surface extérieure, le collecteur d'entrée (42) ayant un orifice d'aération (44), l'orifice d'aération (44) comportant un matériau poreux (48), dans lequel la dimension de pore du matériau poreux est de 0,22 µm à 5,0 µm, **caractérisée en ce que** l'orifice d'aération (44) a une surface superficielle allant de 0,8 cm² à 5,0 cm², et **caractérisée en outre par le fait que** le matériau poreux (48) est collé sur la surface intérieure du collecteur d'entrée (42).

2. Chambre compte-gouttes (12) selon la revendication 1, dans laquelle la dimension de pore du matériau poreux (48) est de 0,45 µm à 5, µm.

3. Chambre compte-gouttes (12) selon la revendication 1 ou 2, dans laquelle la dimension de pore du matériau poreux (48) est d'environ 3 µm.

4. Chambre compte-gouttes (12) selon l'une quelconque des revendications précédentes, dans laquelle le matériau poreux (48) est du polytétrafluoroéthylène expansé (e-PTFE).

5. Chambre compte-gouttes (12) selon l'une quelconque des revendications précédentes, dans laquelle le matériau poreux (48) est un matériau hydrophobe.

6. Chambre compte-gouttes (12) selon l'une quelconque des revendications précédentes, dans laquelle le tube (18) est rigide.

7. Chambre compte-gouttes (12) selon l'une quelconque des revendications précédentes, dans laquelle le tube (18) est de manière générale cylindrique.

8. Chambre compte-gouttes (12) selon l'une quelconque des revendications précédentes, dans laquelle le matériau poreux (48) est formé dans le collecteur d'entrée (42) en créant un trou dans le collecteur d'entrée (42) et en recouvrant le trou à l'aide du matériau poreux (48).

9. Chambre compte-gouttes (12) selon la revendication 7, dans laquelle le matériau poreux (48) est collé sur la surface intérieure du collecteur d'entrée (42) par une technique choisie parmi le groupe à adhésif biocompatible, à piquetage thermique, à soudage aux ultrasons ou à soudage haute fréquence (RF).

10. Système de chambre compte-gouttes pour drainer du fluide cérébrospinal (CSF) à partir d'un cerveau, comportant une chambre compte-gouttes (12) selon l'une quelconque des revendications précédentes ;
un sac de drainage (14) ; et
un robinet d'arrêt reliant le tube (18) au sac de drainage (14) à travers la sortie.
